Europäisches Patentamt

European Patent Office

Office européen des brevets

(11) Veröffentlichungsnummer : **0 078 974**
**B1**

(12) **EUROPÄISCHE PATENTSCHRIFT**

(45) Veröffentlichungstag der Patentschrift :
**12.02.86**

(51) Int. Cl.⁴ : **A 61 F   5/44**

(21) Anmeldenummer : **82109870.4**

(22) Anmeldetag : **26.10.82**

(54) Vorrichtung zur Versorgung einer Ureterostomie.

(30) Priorität : **07.11.81 DE 3144255**

(43) Veröffentlichungstag der Anmeldung :
**18.05.83 Patentblatt 83/20**

(45) Bekanntmachung des Hinweises auf die Patenterteilung : **12.02.86 Patentblatt 86/07**

(84) Benannte Vertragsstaaten :
**AT BE CH DE FR GB IT LI NL SE**

(56) Entgegenhaltungen :
**DE-A- 2 231 396
DE-B- 1 111 776
DE-B- 2 231 397
GB-A- 1 578 020
US-A- 3 055 368
US-A- 3 292 626
US-A- 3 683 918
US-A- 3 837 342**

(73) Patentinhaber : **Beiersdorf Aktiengesellschaft
Unnastrasse 48
D-2000 Hamburg 20 (DE)**

(72) Erfinder : **Sinnen, Marike
Fröbelstrasse 7
D-2080 Pinneberg (DE)**
Erfinder : **Schulte, Dietrich, Dr.
Reichenbergerstrasse 11
D-2080 Pinneberg (DE)**

EP 0 078 974 B1

## Beschreibung

Die Erfindung betrifft eine Vorrichtung zur Versorgung eines künstlichen Harnausgangs, welche sowohl den Ausgang verschließen als auch die Körperausscheidung auffangen kann.

Karzinome, Mißbildungen oder nervenbedingte Funktionsstörungen können es beispielsweise erforderlich machen, einen künstlichen Harnausgang anzulegen. Die Ableitung des Harns über den direkt nach außen geführten Harnleiter hat sich als ungünstig erwiesen, da es hierbei leicht zu einer Stenosierung oder Infektion kommen kann. Vorteilhafter ist es, die Harnableitung über eine ausgeschaltete Dickdarmschlinge vorzunehmen, welche analog der Blase als Reservoir und gleichzeitig durch eine spezielle Operationsmethode als Rückflußsperre für den Harn dient. Die Entleerung dieses Reservoirs erfolgt überlicherweise in einen außen an das Stoma geklebten Beutel, beispielsweise gemäß der DE-BZ-1 111 776, welcher mehrmals am Tage ausgewechselt oder entleert werden muß.

Diese Art der Versorgung eines künstlichen Harnausgangs ist jedoch für den Patienten lästig und hinderlich. Außerdem besteht immer die Schwierigkeit, einen vollkommen dichten Abschluß zu erreichen. Der sich sammelnde Urin löst, meist an einer Stelle beginnend, allmählich die Verklebung des Beutels auf der Haut und kann dann nach außen sickern.

Es wird deshalb angestrebt, mit Hilfe des Colonabschnitts, welcher ein Fassungsvermögen von etwa 100 bis 300 ml besitzt, durch eine neuartige Versorgungs- bzw. Verschlußeinrichtung, die das Tragen eines auffälligen Beutels überflüssig macht, einen weitgehend echten Blasenersatz zu schaffen.

Erfindungsgemäß wird dieses Problem durch eine Vorrichtung aus mindestens einer flüssigkeitsdichten Kunststoffolie gelöst, die auf einer Seite mit einer hautverträglichen Selbstklebemasse beschichtet ist, und in ihrer einfachsten Ausführungsform dadurch gekennzeichnet ist, daß sie Flächenhaft ausgebildet ist und aus nur einer Folienlage aus einer luft- und wasserdampfdurchlässigen, stark dehnbaren Folie aus einem thermoplastischen Polymeren besteht, welche, über das Stoma geklebt, aufgrund ihrer Dehnungsfähigkeit einen weitgehend echten Blasenersatz bildet. Vorteilhafterweise ist sie in der Regel auf der nichtklebenden Seite mit einem schwach haftend beschichteten, stützend wirkenden Hilfsträger abgedeckt.

Das wichtigste Merkmal der Folie ist neben der notwendigen Feuchtigkeitsundurchlässigkeit eine hohe Dehnbarkeit (Reißdehnung), welche im Bereich von mindestens etwa 300 bis 700, vorzugsweise 500 bis 700 %, liegen muß. Wird eine derartige Folie, welche zu ihrer besseren Handhabbarkeit auf einem verstärkend wirkenden Hilfsträger angebracht ist und etwa 10 × 10 bis 15 × 15 cm groß sein sollte, über das Stomaloch geklebt (wonach der Hilfsträger entfernt wird), verschließt sie den Ausgang hermetisch. Füllt sich das Darmvolumen langsam mit Urin und drückt dadurch auf die Verschlußfolie, wobei Drücke von durchschnittlich 0,15 bis zu etwa 0,4 bar auftreten, so gibt die Folie aufgrund ihrer hohen Dehnbarkeit nach, bläht sich vor dem Stomaloch gleich einem kleinen Luftballon auf und schafft ein zusätzliches Volumen von etwa 25 ml zur Aufnahme der Flüssigkeit, ohne daß sich ihre Verklebung auf der Haut löst. Der Patient spürt dann, daß eine Entleerung vorgenommen werden muß.

Besonders geeignet für diesen Zweck sind Polyurethan folien, wie sie beispielsweise in der US-PS 2 871 218 beschrieben sind, aus einem thermoplastischen, weitgehend linearen, d. h. unvernetzten, Polyurethan einer Dicke von etwa 40-150 μm, vorzugsweise etwa 70-80 μm. Derartige Folien sind äußerst schmiegsam und können sich jeder Unebenheit und Bewegung der Haut um das Stoma herum anpassen, was für eine gute Abdichtung erforderlich ist, und geben dem Druck der Flüssigkeit von innen leicht nach, ohne zu reißen (Reißdehnung ca. 500-700 %). Gleichzeitig weisen sie je nach Dicke eine vergleichsweise hohe Wasserdampfdurchlässigkeit (z. B. 350-400 g/m$^2$ · 24 h (gemessen nach Prüf-Norm PPP-T-60 : 38 °C, 95 % rel. Feuchte-Unterschied) für eine Folie von 80 μm Dicke) und Luftdurchlässigkeit auf, so daß auch im verklebten Bereich die Hautfeuchtigkeit abdiffundieren und die Haut atmen kann. Die Folien können durch Extrudieren, im Blasverfahren oder vorzugsweise durch Lösungsmittelgießen hergestellt werden.

Außer diesen besonders vorteilhaften Folien aus Polyurethan lassen sich auch entsprechende Folien aus anderen Materialien, wie z. B. Butadien-Styrol-Blockcopolymeren oder Naturkautschuk verwenden, sofern sie nur die aufgezeigten Eigenschaften erfüllen.

In einer weiteren Ausführungsform ist die erfindungsgemäße Vorrichtung in der Weise ausgestaltet, daß die aufzuklebende Folie mittig eine der Größe des Stoma entsprechende Öffnung aufweist — der Stomadurchmesser beträgt in der Regel ca. 22-25 mm — und eine zweite Folie aus einem stark dehnbaren thermoplastischen Polymeren, welche vorzugsweise etwas kleiner ist als die Basis-Folie, konzentrisch auf der Außenseite dieser Folie mittels einer Schweißnaht befestigt ist.

Eine derartige Vorrichtung besitzt gegenüber der ein fachen Ausführungsform ein erhöhtes Aufnahmevermögen für den Urin entsprechend der Größe der zweiten, sich nunmehr innerhalb des begrenzenden Schweißrings aufblähenden Folie.

Bei dieser Ausführungsform kann als Basis-Folie wieder dieselbe Folie verwendet werden wie bereits bei der einfachen Form beschrieben oder aber auch beispielsweise eine dünne Polyurethanfolie von nur etwa 28 μm Dicke und einer

höheren Wasserdampfdurchlässigkeit von etwa 600-650 g/m² · 24 h. Wesentlich ist, daß die Folie hautfreundlich, gut anschmiegsam und mit der zweiten, lumenbildenden Folie verschweißbar ist. Die zweite Folie ist dagegen dasselbe Material wie bei der einfachen Ausführungsform beschrieben.

In einer weiteren, besonders bevorzugten Ausführungsform der erfindungsgemäßen Vorrichtung ist zwischen der auf den Körper aufzuklebenden Basisfolie und der darauf aufgeschweißten, aufblähbaren zweiten Folie eine dritte, als Rückflußsperre wirkende Folie vorgesehen, die — ebenfalls konzentrisch angebracht — etwas größer als die Öffnung für das Stoma ist und mittels mehrerer einzelner, vorzugsweise geschweißter Befestigungspunkte mit der Basisfolie verbunden ist.

Bei der Füllung der Vorrichtung kann die Flüssigkeit ungehindert unter der Zwischenfolie durch die Kanäle zwischen den Befestigungspunkten in den aufblähbaren Hohlraum einströmen. Beim Abziehen der Vorrichtung jedoch legt sich diese Zwischenfolie durch den Sog verschließend vor die Öffnung für das Stomaloch in der Basisfolie und ermöglicht somit ein weitgehend sauberes Abnehmen der gefüllten Vorrichtung. Die Ventilfolie kann im Prinzip aus den verschiedensten Materialien bestehen sofern sie nur mit der Basisfolie verschweißbar sind, vorzugsweise ist sie jedoch ebenfalls eine dünne thermoplastische Polyurethanfolie von etwa 40 bis 150 μm Dicke wie die beiden anderen Folien.

Da die Vorrichtungen gemäß der Erfindung aufgrund der verwendeten Folie sehr dünn und wenig steif sind, benötigen sie auf ihrer äußeren Seite jeweils einen stützend wirkenden Hilfsträger aus einer festeren Folie oder aus Papier, der gleichzeitig als Applikationshilfe dient. Dieser Hilfsträger ist schwach haftend beschichtet und läßt sich nach dem Anbringen der Vorrichtung leicht wieder abziehen. Vorteilhafterweise ist er hier für mit einer kleinen Anfaßlasche, beispielsweise in Form einer umgeknickten Kante, versehen.

Die der Haut zugewandte Seite der Grundfolie der Vorrichtungen ist mit einer der üblichen gut haftenden Selbstklebemassen beschichtet auf Basis Kautschuk/Harz oder synthetischen Polymeren, vorzugsweise Acrylsäureesterpolymere oder -copolymere, welche zusätzlich noch hydrophile Gruppen und/oder hydrophile Substanzen wie Hydrokolloide enthalten können. Bis zum Gebrauch sollte die Klebeschicht mit einem leicht wieder abziehbaren Schutzblatt aus beispielsweise silikonisiertem Papier abgedeckt sein.

Die Vorrichtungen werden in der Regel direkt auf die Haut aufgeklebt, sie können jedoch auch auf eine zwischengeschaltete sog. Hautschutzfolie, welche längere Zeit um das Stoma herum aufgeklebt bleibt, geklebt werden.

Die Erfindung wird nachstehend anhand der Zeichnungen beispielsweise erläutert.

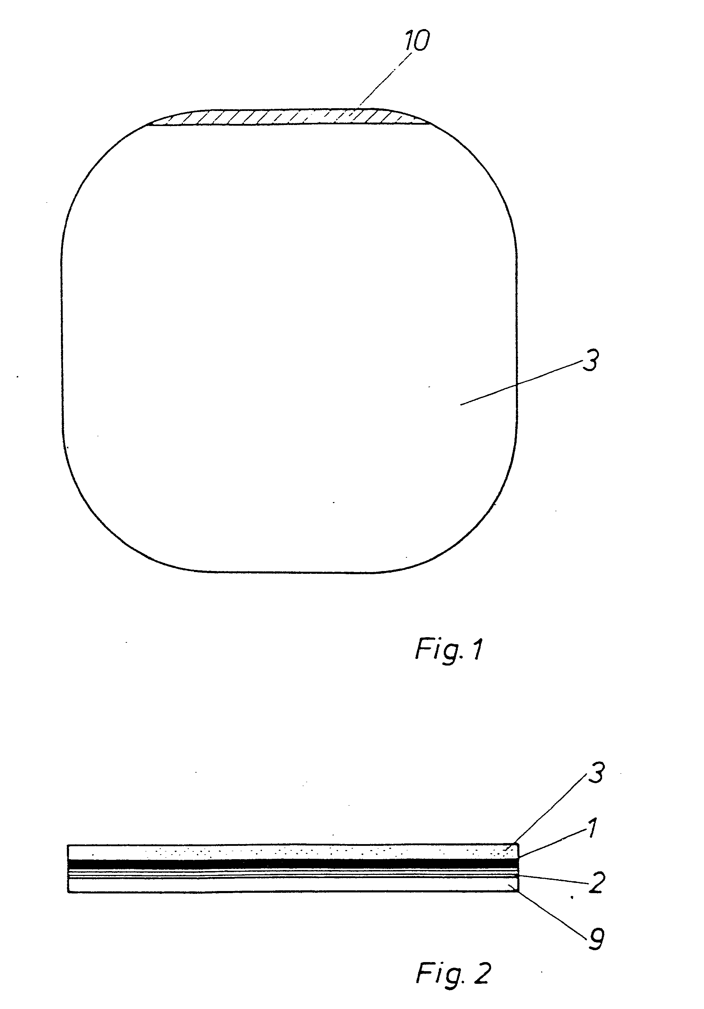

Figur 1 zeigt etwa maßstabsgetreu die erfindungsgemäße Vorrichtung in ihrer einfachen Ausführungsform von oben, wobei 3 den Hilfsträger und 10 die Anfaßlasche bedeuten.

Figur 2 stellt einen Querschnitt durch die Vorrichtung gemäß Figur 1 dar, wobei 1 die hochdehnbare Trägerfolie, 2 die darauf aufgebrachte Selbstklebemasseschicht, 3 den Hilfsträger und 9 das Schutzblatt bedeuten.

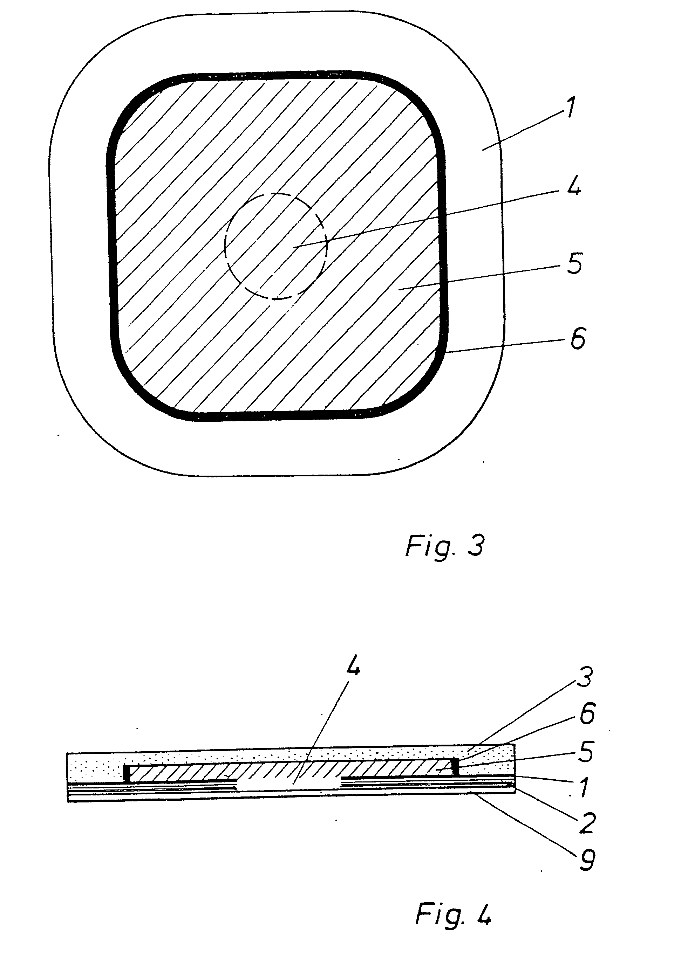

Figur 3 zeigt etwa maßstabsgetreu die erfindungsgemäße Vorrichtung in ihrer zweiten Ausführungsform von oben, wobei der Hilfsträger bereits abgezogen ist. 1 bedeutet wieder die Trägerfolie, 4 die Öffnung für das Stoma, 5 die hochdehnbare zweite Folie und 6 die Schweißnaht.

Figur 4 stellt einen Querschnitt durch die Vorrichtung gemäß Figur 3 dar, wobei 1 die Trägerfolie, 2 die Selbstklebemasseschicht, 3 den Hilfsträger, 4 die Öffnung, 5 die hochdehnbare zweite Folie, 6 die Schweißnaht und 9 das Schutzblatt für die Klebemasseschicht bedeuten.

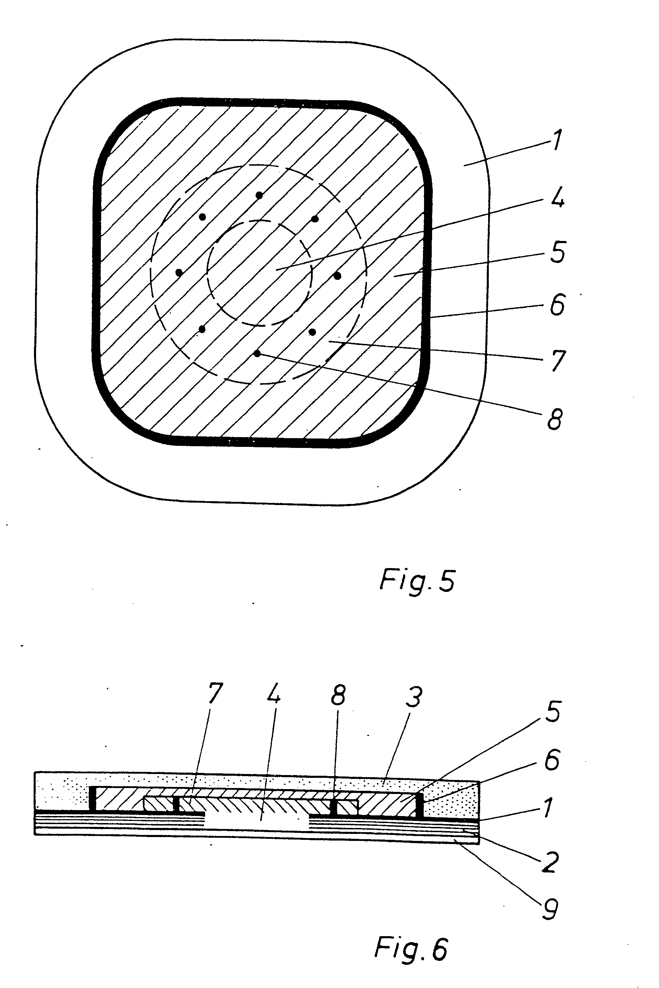

Figur 5 zeigt etwa maßstabsgetreu die erfindungsgemäße Vorrichtung in ihrer dritten Ausführungsform von oben, wobei der Hilfsträger wiederum bereits abgezogen ist. 1 bedeutet wieder die Trägerfolie, 4 die Öffnung für das Stoma, 5 die hochdehnbare zweite Folie, 6 die Schweißnaht, 7 die sich zwischen Basisfolie 1 und lumenbildender Folie 5 befindliche, als Rückflußsperre wirkende dritte Folie (Ventilfolie) und 8 die Befestigungspunkte (Schweißpunkte), mittels welcher die Folie 7 auf der Trägerfolie 1 befestigt ist.

Figur 6 stellt einen Querschnitt durch die Vorrichtung gemäß Figur 5 dar, wobei 1 die Trägerfolie, 2 die Selbstklebemasseschicht, 3 den Hilfsträger, 4 die Öffnung, 5 die hochdehnbare zweite Folie, 6 die Schweißnaht, 7 die Ventilfolie, 8 die Schweißpunkte und 9 das Schutzblatt für die Klebemasseschicht bedeuten.

Die erfindungsgemäßen Vorrichtungen erlauben es dem Patienten eine praktisch vollkommene Kontinenz wieder zu erreichen. Solange die ausgeschiedene Urinmenge das Fassungsvermögen des Darmvolumens nicht übersteigt, schließen sie die Stomaöffnung glatt und dicht anliegend ab. Ist es dem Betroffenen jedoch nicht möglich, rechtzeitig für eine Entleerung zu sorgen, so bieten sie ein ausreichend großes Auffangvolumen ohne den überflüssigen Leerraum und die unangenehme Geräuschbildung wie bei den üblichen Ureterostomiebeuteln.

## Patentansprüche

1. Vorrichtung zur Versorgung eines künstlichen Harnausgangs aus mindestens einer flüssigkeitsdichten Kunststoffolie, die auf einer Seite mit einer hautverträglichen Selbstklebemasse (2) beschichtet ist, dadurch gekennzeichnet, daß sie flächenhaft ausgebildet ist und aus einer Folienlage (1) aus einer luft- und wasserdampfdurchlässigen, stark dehnbaren Folie aus einem thermoplastischen Polymeren besteht, welche, über das Stoma geklebt, aufgrund ihrer Dehnungsfähigkeit einen weitgehend echten Blasenersatz bildet.

2. Vorrichtung gemäß Anspruch 1, dadurch gekennzeichnet, daß die Folie (1) mittig eine etwa der Größe des Stoma entsprechende Öffnung (4) aufweist, eine zweite Folie (5) aus einem stark dehnbaren thermoplastischen Polymeren, welche vorzugsweise etwas kleiner ist als die Folie (1), konzentrisch auf der Außenseite der Folie (1) mittels einer Schweißnaht (6) befestigt ist und über der Folie (1) und (5) gegebenenfalls ein Hilfsträger (3) angebracht ist.

3. Vorrichtung gemäß Anspruch 2, dadurch gekennzeichnet, daß sich zwischen der Folie (1) und der Folie (5) eine weitere Folie (7) mit Ventilfunktion befindet, welche größer als die Öffnung (4) und kleiner als die Folie (5) ist und mittels mehrerer einzelner Befestigungspunkte (8), vorzugsweise Schweißpunkte, konzentrisch auf der Basisfolie (1) befestigt ist.

4. Vorrichtung gemäß den Ansprüchen 1 bis 3, dadurch gekennzeichnet, daß die stark dehnbare Folie (1) und/oder (5) aus einem thermoplastischen Polyurethan besteht.

5. Vorrichtung gemäß Anspruch 4, dadurch gekennzeichnet, daß die Polyurethanfolie (1) und/oder (5) eine Dehnbarkeit von mindestens etwa 500 bis 700 % aufweist.

6. Vorrichtung gemäß mindestens einem der Ansprüche 1 bis 5, dadurch gekennzeichnet, daß die Klebemasseschicht (2) mit einem klebstoffabweisend ausgerüsteten Schutzblatt (9) abgedeckt ist.

7. Vorrichtung gemäß mindestens einem der Ansprüche 1 bis 6, dadurch gekennzeichnet, daß auf der nichtklebenden Seite der Folie (1) bzw. über der Folie (1) und (5) ein schwachhaftend beschichteter, stützend wirkender Hilfsträger (3) angebracht ist.

8. Vorrichtung gemäß Anspruch 7, dadurch gekennzeichnet, daß der Hilfsträger (3) eine Anfaßlasche (10) aufweist.

## Claims

1. Device for the management of an artificial outlet for urine, consisting of at least one liquid-impermeable synthetic film which is coated on one side with a self-adhesive composition (2) which is compatible with skin, characterised in that it is designed in the form of a sheet and consists of one film layer (1) of a highly elastic film which is permeable to air and water vapour, which is composed of a thermoplastic polymer, and which, when stuck over the stoma, forms a substantially genuine replacement bladder because of its elasticity.

2. Device according to Claim 1, characterised in that the film (1) has in the centre an opening (4) which approximately corresponds to the size of the stoma, a second film (5) consisting of a highly elastic thermoplastic polymer, which is preferably somewhat smaller than the film (1), is fixed concentrically on the outside of the film (1) by means of a welded seam (6), and, where appropriate, an auxiliary support (3) is applied over the film (1) and (5).

3. Device according to Claim 2, characterised in that another film (7) with valve function is located between the film (1) and the film (5) and is larger than the opening (4) and smaller than the film (5) and is attached by means of several individual fixing points (8), preferably welding points, concentrically on the basic film (1).

4. Device according to Claims 1 to 3, characterised in that the highly elastic film (1) and/or (5) consists of a thermoplastic polyurethane.

5. Device according to Claim 4, characterised in that the polyurethane film (1) and/or (5) has an elongation at break of at least about 500 to 700 %.

6. Device according to at least one of Claims 1 to 5, characterised in that the layer (2) of adhesive composition is covered with a protective sheet (9) which is rendered adhesive-repellent.

7. Device according to at least one of Claims 1 to 6, characterised in that an auxiliary support (3), which is coated so as to be weakly adhesive and acts as a support, is applied to the non-adhesive side of the film (1) or over the film (1) and (5).

8. Device according to Claim 7, characterised in that the auxiliary support (3) has a gripping flap (10).

## Revendications

1. Dispositif pour l'entretien d'un orifice urinaire artificiel, comprenant au moins un film synthétique imperméable au liquide qui est revêtu sur une face d'une composition auto-adhésive (2) qui est compatible avec la peau, caractérisé en ce qu'il est conçu sous forme d'une feuille et qu'il comprend une couche de film (1) d'un film extrêmement élastique qui est perméable à l'air et à la vapeur d'eau, qui se compose d'un polymère thermoplastique, et qui, lorsqu'il est collé autour du stoma, forme une vessie de remplacement essentiellement authentique grâce à son élasticité.

2. Dispositif selon la revendication 1, caractérisé en ce que le film (1) porte dans son centre une ouverture (4) qui correspond approximativement à la taille du stoma, un second film (5) comprenant un polymère thermoplastique extrêmement élastique, qui est de préférence légèrement plus petit que le film (1), est fixé de façon concentrique à l'extérieur du film (1) au moyen d'un joint soudé (6), et, le cas échéant, un support auxiliaire (3) est appliqué sur le film (1) et (5).

3. Dispositif selon la revendication 2, caractérisé en ce qu'un autre film (7) à fonction de valve est situé entre le film (1) et le film (5) et est plus grand que l'ouverture (4) et plus petit que le film (5) et est attaché au moyen de plusieurs points de fixation individuels (8), de préférence des points de soudure, de façon concentrique sur le film de base (1).

4. Dispositif selon les revendications 1 à 3, caractérisé en ce que le film extrêmement élastique (1) et/ou (5) comprend un polyuréthane thermoplastique.

5. Dispositif selon la revendication 4, caractérisé en ce que le film de polyuréthane (1) et/ou (5) a un allongement à la rupture d'au moins environ 500 à environ 700 %.

6. Dispositif selon l'une au moins des revendications 1 à 5, caractérisé en ce que la couche (2) de composition adhésive est recouverte d'une feuille protectrice (9) qui est rendue répulsive aux adhésifs.

7. Dispositif selon l'une au moins des revendications 1 à 6, caractérisé en ce qu'un support auxiliaire (3), qui est revêtu de façon à être légèrement adhésif et qui joue le rôle de support, est appliqué au côté non adhésif du film (1) ou sur le film (1) et (5).

8. Dispositif selon la revendication 7, caractérisé en ce que le support auxiliaire (3) comporte une patte de préhension (10).

Fig. 1

Fig. 2

Fig. 3

Fig. 4

Fig. 5

Fig. 6